# EUROPÄISCHE PATENTSCHRIFT

(11) **EP 0 442 906 B1**
(45) Veröffentlichungstag und Bekanntmachung des Hinweises auf die Patenterteilung: **16.06.1993**
(21) Anmeldenummer: 89911992.9
(22) Anmeldetag: 09.10.1989
(51) Int. Cl.: A61L 2/06

(54) **STERILISIERBEHÄLTER**
STERILIZER
RECIPIENT DE STERILISATION

(30) Priorität: 10.11.1988 DE 3838099
(43) Veröffentlichungstag der Anmeldung: 28.08.1991
(73) Patentinhaber: AESCULAP AG, D-78501 Tuttlingen (DE)
(72) Erfinder: LOHRER, Walter, D-7200 Tuttlingen (DE); TASCHNER, Wolfgang, D-7200 Tuttlingen (DE)
(74) Vertreter: Böhme, Ulrich Hoeger, Stellrecht & Partner
(86) Internationale Anmeldenummer: EP8901184
(87) Internationale Veröffentlichungsnummer: WO9004981

(56) Entgegenhaltungen:
- DE-C- 3 438 463

## Beschreibung

Die Erfindung betrifft einen Sterilisierbehälter mit einem perforierten Bereich, der durch ein Filter gegenüber dem Behälterinnenraum abdeckbar ist, welches Filter gegen eine Federkraft verschiebbar am Behälter gelagert ist, so daß bei einer Verschiebung des Filters entgegen der Federkraft ein freier, am Filter vorbeiführender Strömungspfad vom Behälterinnern durch den perforierten Bereich hindurch freigegeben wird.

Ein solcher Sterilisierbehälter ist beispielsweise aus der DE-PS 34 38 463 bekannt. Bei diesen Filtern ist der perforierte Bereich durch die Filterschicht dauerhaft abgedeckt und wird nur dann als Sicherheitsmaßnahme freigegeben, wenn der am Filter auftretende Differenzdruck einen bestimmten Wert überschreitet. Dadurch soll eine Beschädigung der Filterlage und des Behälters verhindert werden. Trotzdem bleibt das Filter praktisch während der gesamten Betriebsdauer im Strömungspfad wirksam, so daß auch beim Einströmen des für die Sterilisation notwendigen Wasserdampfes bei einer entsprechenden Verunreinigung desselben das Filter so stark verunreinigt wird, daß es öfter gewechselt werden muß.

Es ist Aufgabe der Erfindung, einen Sterilisierbehälter der gattungsgemäßen Art derart zu verbessern, daß er eine Ventilanordnung erhält, die es möglich macht, mit einer bestimmten Filterlage länger zu arbeiten, ohne daß dabei die Gefahr einer Kontaminierung des Behälterinnern besteht.

Diese Aufgabe wird bei einem Sterilisierbehälter der eingangs beschriebenen Art erfindungsgemäß dadurch gelöst, daß zwischen dem Filter und dem perforierten Bereich eine das gesamte Filter überdeckende Abdeckscheibe parallel zur Verschieberichtung des Filters verschieblich gelagert ist, daß eine das Filter und die Abdeckscheibe voneinander entfernende Feder vorgesehen ist, daß Filter und Abdeckraum in ihrer am stärksten angenäherten Lage abgedichtet aneinanderliegen, daß an der Unterseite eine behälterfeste Abdeckung angeordnet ist, die das Filter überdeckt, daß eine das Filter von der Abdeckung entfernende Feder vorgesehen ist, gegen deren Wirkung das Filter an die Abdeckung abgedichtet anlegbar ist, und daß in der Abdeckung außerhalb des Dichtungsbereichs, innerhalb dessen das Filter abgedeckt ist, Durchströmöffnungen angeordnet sind.

Bei einem solchen Filter ist normalerweise der perforierte Bereich in der Wand oder im Deckel des Sterilisierbehälters von der Filterlage abgedeckt, sowohl die Abdeckscheibe als auch die Abdeckung sind vom Filter entfernt, so daß ein Gasaustausch in der an sich bekannten Weise durch die Perforation und die Filterschicht erfolgen kann.

Tritt Gas mit hoher Geschwindigkeit in den Behälter ein, beispielsweise beim Einströmen des Wasserdampfes zum Sterilisieren, dann verschiebt dieser Dampf die Abdeckscheibe in Richtung auf das Filter, welches dadurch auf seiner Oberseite abgedeckt wird. Gleichzeitig verschiebt die abgedichtet am Filter anliegende Abdeckscheibe auch das Filter und legt dieses abgedichtet an die untere Abdeckung an. Dies führt dazu, daß das Filter auf beiden Seiten abgedichtet abgedeckt und somit vollständig geschützt ist. Durch die Verschiebung des Filters selbst wird die Abdichtung des Filters gegenüber dem perforierten Bereich der Behälterwand oder im Behälterdeckel entfernt, so daß nunmehr der Gasaustausch außerhalb der abgedichteten Bereiche erfolgen kann, ohne daß dabei eine Filterung eintritt. Dies ist jedoch beim Einlaß des Wasserdampfes auch nicht notwendig, da der Wasserdampf selbst erst die Sterilisation hervorruft.

In dieser zweiten Betriebsphase ist also die Baueinheit aus Abdeckscheibe, Filter und unterer Abdeckung aus dem eigentlichen Strömungsweg entfernt, während in der ersten Betriebsphase das Filter im Strömungspfad liegt und beidseitig von Abdeckscheibe beziehungsweise unterer Abdeckung freigegeben wird.

Auf diese Weise wird das Filter nur in einem Teil des Betriebsablaufes in den Strömungspfad eingeschaltet, während in einer anderen Betriebsphase, in der eine besonders starke Verschmutzung des Filters zu befürchten wäre, das Filter sich nicht im Strömungspfad befindet. Dies erlaubt eine wesentlich längere Verwendung eines bestimmten Filters bei Aufrechterhaltung derselben Betriebssicherheit.

Bei einem bevorzugten Ausführungsbeispiel der Erfindung ist vorgesehen, daß die Abdeckscheibe längs ihres Verschiebeweges an einem Anschlag anschlägt, so daß zwischen der Abdeckscheibe und dem perforierten Bereich ein Strömungspfad freibleibt.

Es kann weiterhin vorgesehen sein, daß der perforierte Bereich von einem zylindrischen Stutzen umgeben ist, an dessem freien Rand eine den Filterbereich des Filters umgebende Dichtung anliegt, wenn das Filter durch die das Filter von der Abdeckung entferndende Feder in seine perforationsnahe Endstellung verschoben ist.

Es ist dabei günstig, wenn der Stutzen die Abdeckscheibe im Abstand umgibt.

Zur Abdichtung der Abdeckscheibe gegenüber dem Filter kann vorgesehen sein, daß am Filter und an der Abdeckscheibe eine den Filterbereich umgebende Dichtung beziehungsweise ein Dichtsitz angeordnet sind.

In gleicher Weise können an dem Filter und an der Abdeckung eine den Filterbereich umgebende Dichtung beziehungsweise ein Dichtsitz angeordnet sein. Die Dichtungen sind dabei vorzugsweise in Nuten eingelegte Silikondichtungen.

Eine besonders kompakte Konstruktion ergibt sich, wenn zwischen Filter und Abdeckscheibe sowie zwischen Filter und Abdeckung je eine Schraubenfeder eingelegt ist. Diese Schraubenfedern verschieben die Abdeckscheibe und das Filter in die vom Filter beziehungsweise von der unteren Abdeckung entfernte Position. Sie befinden sich in dem abgedichteten Hohlraum, der sich ergibt, wenn die Abdeckscheibe kräftig in Richtung auf die untere Abdeckung verschoben wird.

Besonders vorteilhaft ist es, wenn das Filter und die Abdeckscheibe auf einem zentralen, senkrecht zum perforierten Bereich verlaufenden Zapfen verschieblich gelagert sind, der vorzugsweise an der Abdeckung gehalten ist.

Die Abdeckung kann mittels einer Bajonetteverriegelung lösbar an der den perforierten Bereich aufweisenden Wand des Behälters gehalten sein, so daß durch Abnahme der Abdeckung die gesamte Filterkonstruktion von der perforierten Wand oder dem perforierten Deckel abgenommen werden kann. Dies erleichtert die Reinigung und gegebenenfalls auch das Auswechseln der Filterlage.

Bei einer besonders bevorzugten Ausführungsform weist die Abdeckung eine zylindrische Seitenwand auf, die sich bis zu der den perforierten Bereich tragenden Wand erstreckt und die Abdeckscheibe und Filter sowie gegebenenfalls den Stutzen außen umschließt. Diese Abdeckung bildet somit ein Gehäuse für die gesamte Filter- und Ventilanordnung.

Die nachfolgende Beschreibung bevorzugter Ausführungsformen der Erfindung dient im Zusammenhang mit der Zeichnung der näheren Erläuterung.

Es zeigen:
- Figur 1:: eine Querschnittsansicht eines Filterventils mit in den Strömungspfad eingeschaltetem Filter und
- Figur 2:: eine Ansicht ähnlich Figur 1 mit aus dem Strömungspfad entferntem Filter.

Die in der Zeichnung dargestellte Filter-Ventilanordnung kann grundsätzlich an jeder Wand eines Sterilisierbehälters angeordnet sein, die einen perforierten Bereich 1 aufweist. Im vorliegenden Beispiel ist der perforierte Bereich 1 in der oberen Wand 2 eines in der Zeichnung nur teilweise dargestellten Deckels angeordnet. Der perforierte Bereich 1 kann beispielsweise eine kreisförmige Fläche einnehmen.

Bei dem dargestellten Ausführungsbeispiel wird dieser perforierte Bereich 1 im Abstand von einer den perforierten Bereich 1 nur über einen Teil seines Umfangs umgebenden Halteleiste 3 umschlossen, die einen ersten, flächig an der Wand 2 anliegenden und in diesem Bereich an der Wand 2 gehaltenen Bereich 4 und einen auf den perforierten Bereich 1 hin gerichteten, gegenüber dem Bereich 4 seitlich versetzten und somit einen Abstand zwischen sich und der Wand 2 einhaltenden zweiten Bereich 5 aufweist. Ein kreiszylindrisches Gehäuse 6 greift mit seinem radial nach außen umgebogenen oberen Rand 7 in den Zwischenraum 8 zwischen dem Bereich 5 der Halteleiste 3 und der Wand 2, so daß das Gehäuse 6 auf diese Weise an der Wand 2 gehalten ist. Auch der Rand 7 erstreckt sich nur über einen Teil des Umfangs des zylindrischen Gehäuses 6, so daß durch Verdrehen des Gehäuses 6 um die Zylinderachse der Rand 7 aus dem Zwischenraum 8 herausgedreht werden kann, bis das Gehäuse 6 von der Wand 2 abnehmbar ist.

Die der Wand 2 abgewandte untere Stirnwand 9 ist im wesentlichen eben ausgebildet und verschließt das zylindrische Gehäuse 6 weitgehend. Der zentrale Teil der Stirnwand 9 wird begrenzt durch eine Sicke 10, die im Querschnitt die Form von aneinander anschließenden, entgegengerichteten Halbwellen aufweist. Radial außerhalb der Sicke 10 sind längs des Umfanges der Stirnwand 9 eine größere Anzahl von Öffnungen 11 vorgesehen.

Auf der Zylinderachse des Gehäuses 6 trägt die Stirnwand 9 einen senkrecht zur Wand 2 verlaufenden Lagerzäpfen 12, der in geringem Abstand von der Wand 2 in Form eines erweiterten Kopfes 13 endet. Der Lagerzapfen kann beispielsweise in die Stirnwand 9 eingeschraubt sein, so daß der Lagerzapfen bei Bedarf von der Stirnwand 9 getrennt werden kann.

Oberhalb der als Abdeckung wirkenden Stirnwand 9 ist eine Filterscheibe 14 mit einer zentralen Öffnung 15 angeordnet, durch welche der Lagerzapfen 12 hindurchtritt. Im Durchtrittsbereich liegt die Filterscheibe 14 mit einem nach oben abgebogenen Ringflansch 16 abdichtend an dem Lagerzapfen 12 an. Dieser zentrale Durchtrittsbereich des Lagerzapfens 12 ist von einem ringförmigen Filter 17 umgeben, welches sich in radialer Richtung etwa bis zum Beginn der Sicke 10 in der Stirnwand 9 erstreckt. Das Filter kann ein Metallsieb oder ein Glasfasersieb sein, es kann auch aus Kunststoff-Fasern bestehen.

Der äußere Rand des Filters 17 wird umgeben von zwei nebeneinanderliegenden Ringnuten 18 und 19, die durch Einprägung in der Filterscheibe 14 ausgebildet sind. Die innere Ringnut 18 öffnet sich nach unten und befindet sich oberhalb des äußeren Teils der Sicke 10 in der Stirnwand 9. Die äußere Ringnut 19 schließt sich unmittelbar an die innere Ringnut 18 an und ist nach oben hin geöffnet. In beiden Ringnuten 18 und 19 ist jeweils eine Silikondichtung 20 beziehungsweise 21 eingelegt. Ringnut 18 ist an ihrem Boden noch einmal zusätzlich ausgebuchtet, so daß an der Oberseite der Filterscheibe 14 im Bereich der inneren Ringnut 18 eine umlaufende Dichtkante 22 entsteht.

Eine Schraubenfeder 23 ist zwischen Stirnwand 9 und Filterscheibe 14 den Lagerzapfen 12 konzentrisch umgebend so eingelegt, daß sie sich mit ihrem unteren Teil in dem inneren Abschnitt der Sicke 10 abstützt, mit dem oberen Teil an der Unterseite der Filterscheibe 14. Diese Schraubenfeder 23 verschiebt die Filterscheibe 14 auf dem Lagerzapfen 12 in Richtung auf die Wand 2, bis die Silikondichtung 21 in der äußeren Ringnut 19 am freien Rand 24 eines Stutzens 25 anliegt, der mit einem radial nach außen abstehenden Flansch 26 an der Unterseite der Wand 2 gehalten ist und den perforierten Bereich 1 konzentrisch umgibt.

In dem von dem Stutzen 25 umschlossenen Raum befindet sich eine Abdeckscheibe 27 mit einer zentralen Öffnung 28, durch die ebenfalls der Lagerzapfen 12 hindurchtritt. Die Abdeckscheibe 27 liegt im Bereich der Öffnung 28 mit einem nach unten umgebogenen Flansch 29 dichtend am Lagerzapfen an. Die Abdeckscheibe 27 ist im wesentlichen eben und ohne Öffnungen ausgebildet, sie trägt an ihrem äußeren Rand eine nach unten offene Ringnut, die sich genau über der inneren Ringnut 18 der Filterscheibe 14 befindet und ebenfalls eine Silikondichtung 31 aufnimmt. Zwischen der Filterscheibe 14 und der Abdeckscheibe 27 ist eine weitere, den Lagerzapfen 12 konzentrisch umgebende Schraubenfeder 32 angeordnet, die die Abdeckscheibe gegenüber der Filterscheibe nach oben gegen den erweiterten Kopf 13 des Lagerzapfens 12 drückt.

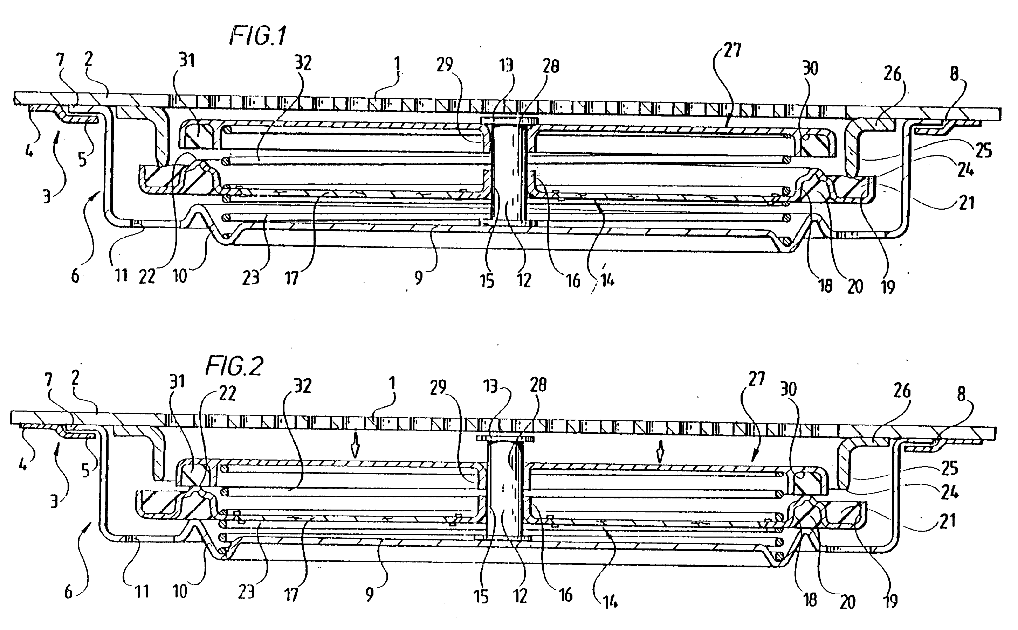

Im normalen Betrieb, wenn kein starker Gasstrom durch den perforierten Bereich 1 hindurchtritt (Figur 1), ist die Filterscheibe 14 durch die untere Schraubenfeder 23 abdichtend gegen den unteren Rand 24 des Stutzens 25 und die Abdeckscheibe 27 durch die obere Schraubenfeder 32 gegen den Kopf 13 des Lagerzapfens 12 gedrückt. Es ergibt sich dadurch ein Strömungspfad durch die Öffnungen des perforierten Bereiches 1, an der Außenseite der Abdeckscheibe 27 vorbei in den Zwischenraum zwischen Abdeckscheibe 27 und Filterscheibe 14. Der Gasstrom muß in diesem Falle durch das Filter 17 der Filterscheibe 14 hindurchtreten, da die Filterscheibe 14 abdichtend gegen den Stutzen 25 gedrückt ist. Nach dem Durchtritt durch das Filter tritt die Gasströmung durch die Öffnungen 11 in das Behälterinnere ein.

Beim Abkühlen des Behälterinhalts, bei dem im Inneren ein Unterdruck entsteht, wird auf diese Weise Luft aus der Umgebung angesaugt, die durch das Filter 17 sicher gereinigt wird. Auch nach dem Sterilisieren noch im Behälterinneren enthaltener Wasserdampf kann auf umgekehrtem Wege durch das Filter 17 nach außen gelangen, ohne daß dabei die Gefahr besteht, daß eine Verunreinigung ins Behälterinnere gelangt.

Wenn dagegen eine sehr große Druckdifferenz zwischen außen und innen auftritt und dadurch eine sehr starke, nach innen gerichtete Strömung entsteht, wie dies beispielsweise beim Eintreten des Wasserdampfes zu Beginn des Sterilisiervorgangs der Fall ist, dann wird dadurch die Abdeckscheibe 27 in Richtung auf die Stirnwand 9 verschoben, bis ihre Silikondichtung 31 abdichtend an der Dichtkante 22 der Filterscheibe 14 anliegt und somit das Filter 17 auf der Oberseite dicht abdeckt. Gleichzeitig drückt die Abdeckscheibe 27 die Filterscheibe 14 bei einem weiteren Verschieben in Richtung auf die Stirnwand 9 gegen den äußeren Teil der Sicke 10, die somit an der Silikondichtung 20 dichtend zur Anlage gelangt und damit das Filter 17 von der Unterseite her dicht abschließt (Figur 2). In dieser Stellung wird das Filter also durch die Abdeckscheibe 27 einerseits und durch die als Abdeckung wirkende Stirnwand 9 andererseits in einem abgedichteten Raum eingeschlossen, so daß das Filter nicht mehr von der Gasströmung durchströmt wird. Das eintretende Gas gelangt vielmehr außen an der Abdeckscheibe 27 und der Filterscheibe 14 vorbei unmittelbar zu den Öffnungen 11 in der Stirnwand 9. Da ein so starkes Einströmen normalerweise nur vor dem Sterilisieren beim Einleiten des Wasserdampfes auftritt, ist es nicht schädlich, wenn dabei der Wasserdampf ungefiltert eintreten kann. Sobald nämlich der Eintritt des Wasserdampfes vollzogen ist, schieben die beiden Schraubenfedern 23 und 32 die Filterscheibe 14 und die Abdeckscheibe 27 wieder in die in Figur 1 dargestellte Ausgangsstellung, in der das Behälterinnere durch das Filter 17 abgeschlossen ist.

## Patentansprüche

1. Sterilisierbehälter mit einem perforierten Bereich, der durch ein Filter gegenüber dem Behälterinnenraum abdeckbar ist, welches Filter gegen eine Federkraft verschiebbar am Behälter gelagert ist, so daß bei einer Verschiebung des Filters entgegen der Federkraft ein freier, am Filter vorbeiführender Strömungspfad vom Behälterinneren durch den perforierten Bereich hindurch freigegeben wird;
**dadurch gekennzeichnet**, daß
zwischen dem Filter (17) und dem perforierten Bereich (1) eine das gesamte Filter (17) überdeckende Abdeckscheibe (27) parallel zur Verschieberichtung des Filters (17) verschieblich gelagert ist, daß eine das Filter (17) und die Abdeckscheibe (27) voneinander entfernende Feder (32) vorgesehen ist, daß Filter (17) und Abdeckscheibe (27) in ihrer am stärksten angenäherten Lage abgedichtet aneinanderliegen, daß an der Unterseite eine behälterfeste Abdeckung (9) angeordnet ist, die das Filter (17) überdeckt, daß eine das Filter (17) von der Abdeckung (9) entfernende Feder (23) vorgesehen ist, gegen deren Wirkung das Filter (17) an die Abdeckung (9) abgedichtet anlegbar ist, und daß in der Abdeckung (9) außerhalb des Dichtungsbereiches, innerhalb dessen das Filter (17) abgedeckt ist, Durchströmöffnungen (11) angeordnet sind.

2. Behälter nach Anspruch 1, dadurch gekennzeichnet, daß die Abdeckscheibe (27) längs ihres Verschiebeweges an einen Anschlag (13) anschlägt, so daß zwischen der Abdeckscheibe (27) und dem perforierten Bereich (1) ein Strömungspfad freibleibt.

3. Behälter nach einem der Ansprüche 1 oder 2, dadurch gekennzeichnet, daß der perforierte Bereich (1) von einem zylindrischen Stutzen (25) umgeben ist, an dessen freiem Rand (24) eine den Filterbereich des Filters (17) umgebende Dichtung (20) anliegt, wenn das Filter (17) durch die das Filter (17) von der Abdeckung (9) entfernende Feder (23) in seine perforationsnahe Endstellung verschoben ist.

4. Behälter nach Anspruch 3, dadurch gekennzeichnet, daß der Stutzen (25) die Abdeckscheibe (27) im Abstand umgibt.

5. Behälter nach einem der voranstehenden Ansprüche, dadurch gekennzeichnet, daß am Filter (17) und an der Abdeckscheibe (27) eine den Filterbereich umgebende Dichtung (31) beziehungsweise ein Dichtsitz (22) angeordnet sind.

6. Behälter nach einem der voranstehenden Ansprüche, dadurch gekennzeichnet, daß an dem Filter (17) und an der Abdeckung (9) eine den Filterbereich umgebende Dichtung (20) beziehungsweise ein Dichtsitz (10) angeordnet sind.

7. Behälter nach einem der Ansprüche 5 oder 6, dadurch gekennzeichnet, daß die Dichtungen (20, 21, 31) in Nuten (18, 19, 30) eingelegte Silikondichtungen sind.

8. Behälter nach einem der voranstehenden Ansprüche, dadurch gekennzeichnet, daß zwischen Filter (17) und Abdeckscheibe (27) sowie zwischen Filter (17) und Abdeckung (9) je eine Schraubenfeder (32, 23) eingelegt ist.

9. Behälter nach einem der voranstehenden Ansprüche, dadurch gekennzeichnet, daß das Filter (17) und die Abdeckscheibe (27) auf einem zentralen, senkrecht zum perforierten Bereich (1) verlaufenden Zapfen (12) verschieblich gelagert sind.

10. Behälter nach Anspruch 9, dadurch gekennzeichnet, daß der Zapfen (12) an der Abdeckung (9) gehalten ist.

11. Behälter nach einem der voranstehenden Ansprüche, dadurch gekennzeichnet, daß die Abdeckung (9) mittels einer Bajonettverriegelung (5, 7) lösbar an der den perforierten Bereich (1) aufweisenden Wand (2) des Behälters gehalten ist.

12. Behälter nach einem der voranstehenden Ansprüche, dadurch gekennzeichnet, daß die Abdeckung (9) eine zylindrische Seitenwand aufweist, die sich bis zu der den perforierten Bereich (1) tragenden Wand (2) erstreckt und Abdeckscheibe (27) und Filter (17) sowie gegebenenfalls den Stutzen (25) außen umschließt.

## Claims

1. Sterilizing container comprising a perforated region adapted to be covered in relation to the interior of the container by a filter mounted on the container for displacement contrary to the force of a spring such that a displacement of the filter contrary to the force of the spring results in the opening of a free flow path directed past the filter from the interior of the container through the perforated region,
characterized in that a cover plate (27) covering the entire filter (17) is mounted between the filter (17) and the perforated region (1) for displacement parallel to the direction of displacement of the filter (17), that a spring (32) is provided for moving the filter (17) and the cover plate (27) apart from one another, that filter (17) and cover plate (27) contact one another in a sealing manner in their position of closest proximity, that a cover (9) fixed to the container is arranged on the underside thereof and covers the filter (17), that a spring (23) is provided for moving the filter (17) away from the cover (9), the filter (17) being adapted to be applied in a sealing manner to the cover (9) contrary to the action of said spring, and that flow openings (11) are arranged in the cover (9) outside the sealing region within which the filter (17) is covered.

2. Container as defined in claim 1, characterized in that the cover plate (27) strikes against a stop (13) along its path of displacement such that a flow path remains open between the cover plate (27) and the perforated region (1).

3. Container as defined in either of claims 1 or 2, characterized in that the perforated region (1) is surrounded by a cylindrical connecting piece (25), a seal (20) surrounding the filter region of the filter (17) abutting on the free edge (24) of said connecting piece when the filter (17) is moved into its end position near the perforation by the spring (23) moving the filter (17) away from the cover (9).

4. Container as defined in claim 3, characterized in that the connecting piece (25) surrounds the cover plate (27) in spaced relation thereto.

5. Container as defined in any of the preceding claims, characterized in that a seal (31) surrounding the filter region and a seal seat (22) are arranged on the filter (17) and the cover plate (27), respectively.

6. Container as defined in any of the preceding claims, characterized in that a seal (20) surrounding the filter region and a seal seat (10) are arranged on the filter (17) and the cover (9), respectively.

7. Container as defined in either of claims 5 or 6, characterized in that the seals (20, 21, 31) are silicon seals inserted in grooves (18, 19, 30).

8. Container as defined in any of the preceding claims, characterized in that a helical screw (32, 23) is inserted between filter (17) and cover plate (27) as well as between filter (17) and cover (9).

9. Container as defined in any of the preceding claims, characterized in that the filter (17) and the cover plate (27) are mounted for displacement on a central pin (12) extending perpendicular to the perforated region (1).

10. Container as defined in claim 9, characterized in that the pin (12) is held on the cover (9).

11. Container as defined in any of the preceding claims, characterized in that the cover (9) is releasably held by a bayonet catch means (5, 7) on the wall (2) of the container comprising the perforated region (1).

12. Container as defined in any of the preceding claims, characterized in that the cover (9) has a cylindrical side wall extending as far as the wall (2) bearing the perforated region (1) and externally enclosing cover plate (27) and filter (17) as well as, when necessary, the connecting piece (25).

## Revendications

1. Récipient de stérilisation comprenant une région perforée qui peut être isolée du volume intérieur du récipient par un filtre, lequel filtre est monté sur le récipient pour se déplacer en translation à l'encontre d'une force élastique, de telle manière que le déplacement du filtre à l'encontre de la force élastique dégage un trajet d'écoulement libre partant du volume intérieur du récipient contournant le filtre et traversant la région perforée,
caractérisé en ce que qu'entre le filtre (17) et la région perforée (1), est monté mobile en translation un disque de recouvrement (27) qui recouvre tout le filtre (17), et qui est mobile en translation parallèlement à la direction de la translation du filtre, qu'il est prévu un ressort (32) tendant à éloigner le filtre (17) et le disque de recouvrement (27) l'un de l'autre, que le filtre (17) et le disque de recouvrement (27) sont appuyés l'un contre l'autre à joint étanche dans leur position la plus fortement rapprochée, qu'au droit de la face inférieure, est disposé un recouvrement (9) solidaire du récipient, qui recouvre le filtre (17), qu'il est prévu un ressort (23) tendant à éloigner le filtre du recouvrement (9), à l'encontre de l'action duquel le filtre (17) peut être appliqué à joint étanche contre le recouvrement, et que des ouvertures de traversée (11) sont prévues dans le recouvrement (9) à l'extérieur de la région d'étanchéité dans laquelle le filtre (17) est recouvert.

2. Récipient selon la revendication 1, caractérisé en ce que le disque de recouvrement (27) bute contre une butée (13) sur son trajet de translation, de sorte qu'il subsiste un trajet d'écoulement entre le disque de recouvrement (27) et la région perforée (1).

3. Récipient selon une des revendications 1 et 2, caractérisé en ce que la région perforée (1) est entourée d'un collet cylindrique (25) contre le bord libre (24) duquel s'appuie une garniture d'étanchéité (20) qui entoure la région filtrante du filtre (17) lorsque le filtre (17) est repoussé à sa position extrême proche de la perforation par le ressort (23) qui éloigne le filtre (17) du recouvrement (9).

4. Récipient selon la revendication 3, caractérisé en ce que le collet (25) entoure le disque de recouvrement (27) à un certain écartement.

5. Récipient selon une des revendications précédentes, caractérisé en ce que, sur le filtre (17) et sur le disque de recouvrement (27) sont disposés respectivement une garniture d'étanchéité (31) qui entoure la région filtrante et une portée d'étanchéité (22).

6. Récipient selon une des revendications précédentes, caractérisé en ce que, sur le filtre (17) et sur le recouvrement (9) sont disposées respectivement une garniture d'étanchéité (20) qui entoure la région filtrante et une portée d'étanchéité (10).

7. Récipient selon une des revendications 5 et 6, caractérisé en ce que les garnitures d'étanchéité (20, 21, 31) sont des garnitures d'étanchéité en silicone encastrées dans des gorges (18, 19, 30).

8. Récipient selon une des revendications précédentes, caractérisé en ce que des ressorts hélicoïdaux (32, 23) sont intercalés respectivement entre le filtre (17) et le disque de recouvrement (27) et entre le filtre (17) et le recouvrement (9).

9. Récipient selon une des revendications précédentes, caractérisé en ce que le filtre (17) et le disque de recouvrement (27) sont montés coulissants sur un tourillon central (12) s'étendant perpendiculairement à la région perforée (1).

10. Récipient selon la revendication 9, caractérisé en ce que le tourillon (12) est fixé au recouvrement (9).

11. Récipient selon une des revendications précédentes, caractérisé en ce que le recouvrement (9) est fixé de façon démontable à la paroi (2) du récipient qui présente la région perforée (1) au moyen d'un verrouillage à baïonnette (5, 7).

12. Récipient selon une des revendications précédentes, caractérisé en ce que le recouvrement (9) présente une paroi latérale cylindrique qui s'étend jusqu'à la paroi (2) qui porte la région perforée, et qui entoure extérieurement le disque de recouvrement (27) et le filtre (17) ainsi qu'éventuellement le collet (25).
